(19)

Europäisches Patentamt

European Patent Office

Office européen des brevets

(11) **EP 3 612 963 B1**

(12) **EUROPEAN PATENT SPECIFICATION**

(45) Date of publication and mention of the grant of the patent:
**12.05.2021 Bulletin 2021/19**

(21) Application number: **17781217.9**

(22) Date of filing: **18.04.2017**

(51) Int Cl.:
**G16H 40/63** *(2018.01)*     **G16H 30/40** *(2018.01)*

(86) International application number:
**PCT/TR2017/050150**

(87) International publication number:
**WO 2018/194525 (25.10.2018 Gazette 2018/43)**

(54) **BIOCHEMICAL ANALYSER BASED ON A MACHINE LEARNING ALGORITHM USING TEST STRIPS AND A SMARTDEVICE**

BIOCHEMISCHER ANALYSATOR AUF DER BASIS EINES MASCHINENLERNALGORITHMUS UNTER VERWENDUNG VON TESTSTREIFEN UND EINER INTELLIGENTEN VORRICHTUNG

ANALYSEUR BIOCHIMIQUE BASÉ SUR UN ALGORITHME D'APPRENTISSAGE AUTOMATIQUE UTILISANT DES BANDELETTES D'ESSAI ET UN DISPOSITIF INTELLIGENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.02.2020 Bulletin 2020/09**

(73) Proprietor: **Yeditepe Universitesi
Istanbul (TR)**

(72) Inventors:
• **KESKIN, Ali Umit
Atasehir/Istanbul (TR)**
• **HAMAMCI, Andac
Atasehir/Istanbul (TR)**

(74) Representative: **Dericioglu, E. Korhan
Ankara Patent Bureau
Kavaklidere Mahallesi Bestekar Caddesi No: 10
06680 Cankaya, Ankara (TR)**

(56) References cited:
**WO-A1-2013/116831     US-A1- 2008 309 929
US-A1- 2016 048 739**

**EP 3 612 963 B1**

## Description

## Field of the Invention

Present invention relates generally to the field of clinical diagnostics. Present invention relates specifically to a diagnostic system and methods for obtaining consistent measurements and analyses, using dipsticks (test strips) in a smart device platform.

## Background of the Invention

Portable computing and communication devices, such as smartphones and tablets (smart device), have penetrated everybody's daily activities, and they also provide considerable read-out, data storage, and connectivity power. New advantages of this technology can be realized in many fields of healthcare.

The advanced features and massive uptake of cell phones (70% of whom reside in developing countries) make them highly suitable for personalized point-of-care testing (POC) and the decentralization of health-care management, particularly in remote, resource-deficient private and public settings.

A Smartphone can run specific application programs which are based on operating systems, such as Android, iOS, or Windows Mobile.

On the other hand, dipsticks (test strips) are disposable modular test platforms that can be used in conjunction with a smartphone. They contain test and calibration regions where the latter region is used as the reference to the selected parameter of the tested body fluid (analyte). Dipsticks can be sensitive to a chemical, or enzymatic colorimetric reactions. Dipsticks may contain multiple reagent pads that change color on contacting an analyte.

Each pad on a dipstick uses an absorbent material that has been impregnated with a mixture of dyes that shift color when contacted to the solution being tested. In routine clinical laboratory practice, this resulting color can be visually compared against a color chart to estimate the amount of fluid component or its characteristic value (such as pH).

For example, a test strip (hereafter TS) for urine analysis may consist of multiple colorimetric reagent pads compared with respective color scales 1-2 minutes following its immersion in urine sample (exposure of TS). The glucose, Red blood cells, pH, protein, urobilinogen, bilirubin, ketone, specific gravity, nitrites, ascorbic acid, and leucocytes concentration in urine can be analyzed by using conventional urine analysis procedure. The test results can then be interpreted by a physician.

Although evaluation of the color changes can be done by the naked eye, this may cause uncertainties due to personal subjectivity and the environment (lighting) conditions. Routine clinical practice of this approach involves the use of photometric devices which makes the test more accurate than visual assessment against printed color scales (e.g., Siemens Clinitek Status Analyzer, Urisys by Roche, SD Urometer by Standard Diagnostics, Mission Analyzer by Acon Laboratories).

Operating principles of such laboratory and mobile analyzers are needed to be well understood before attempting to design a smart-device based colorimetric analyzer. For example, Lappe et al, in US Patent No 6514461 B1, "System for automatically testing a fluid specimen", (04 Feb 2003) teaches a system which automates sampling of liquid followed by imaging of test strips with camera. The system described in US Patent No 5408535 by Howard et.al. relies on video imaging of standard test strips with an automation system for use in a laboratory environment. A US Patent Application, No. US 8797536 B2 (by D. S. Lee, et.al., "Portable digital reader for urinalysis", Pub. Date 5 August 2014) uses a three-color LED as a light source. Light is cast on a biochip for detection, a silicon sensor is used to analyze intensity and color of light reflected by the biochip. Apparatus described in Patent WO 2015042523 A1 by Ying Chi, et.al. includes a cavity for accommodating a medical specimen to be detected, a white light source, and multiple digital camera units for photographing the specimen to be detected. The camera units each include optics with positive refractive power and an image sensor smaller than 1/3 inches.

US Patent application no 2012/0244624 A1, Sep. 27, 2012, "Analyzing Method and System for Test Strips" by H. Hsiao teaches about an exemplary test strip reading and analyzing system which includes a test strip reading unit, a mobile device and a remote computing apparatus. The mobile device captures a test-strip image and a transmission module electrically coupled to the image capture module transmits it to remote computing apparatus. The remote computing apparatus analyzes the test-strip image and generates a test report which is then transmitted back to the mobile device.

Chen et al., in US Patent 8506901, (2013) describe a liquid sample cup with a lid that incorporates color strips activated by inverting the cup and then the outside of the lid is imaged by mobile device and processed This system fails to integrate test and calibration into the container. It does not control for the impact of ambient light.

US Patent 20150359458 A1 by D. Erickson et.al., (Publication date of December 17, 2015, titled "Smartphone-Based Apparatus and Method for Obtaining Repeatable, Quantitative Colorimetric Measurement") teaches about taking a color image of the test strip following its reaction with the analyte by illuminating the rear surface of the dipstick. This requires a separate removable attachment coupled to the smartphone, which is to be adapted to receive dipsticks. The accessory includes a light diffuser and/or a light-diffusing pathway so as to ensure proper illumination of a test strip, which further requires to be substantially light-tight when a dipstick is inserted therein, so as to enable consistent internal illumination conditions independently of any external conditions. The light source is one of an internal smartphone flash source and an external LED source. The designed-in optical pathway in the housing of the attachment comprises a wall that creates an indirect optical path between the external light source resident in the smartphone and a resident smartphone camera in the smartphone to which the smartphone attachment can be connected.

A patent WO2015038717 A1 owned by N. Agarwal, et.al. (publication date: 19 Mar 2015, Title: "System for diagnostic testing of liquid samples") discloses a system for analysis of a fluid comprising an opaque sample container with color changing indicators within the container that respond to exposure to the urine sample. The test pads are mounted on a test card. The test card has a uniform base color. A smartphone images the test card, and standardize the color values. Standardization of color values is achieved using the regions selected from uniform base color of test card to compensate for spatial variation in light intensity and by using an additional color calibration reference attached to the test card to compensate for difference in color sensitivity of the camera and fluctuations in wavelength distribution of the flash. Smartphone camera flash provides illumination while the opaque container shields against variations from ambient light. A lid for the container provides a place to position the smartphone for imaging and also includes a diffuser for the camera flash to make the illumination more uniform. The test card can be mounted inside the container to reduce specular reflection. The change in color is used to calculate urine tests results using a correlation model implemented in software.

A urinalysis system, uChek (Biosense Technologies Private Ltd., India) which is currently available for sale in India only (http://www.biosense.in/uchek.html) uses a smartphone (iPhone 4, 4S and 5) camera for imaging of the test strip (Multistix by Siemens) on the principle of reflectance photometry alongside color calibration patches. It can perform calculations such as determining the albumin to creatinine ratio in urine as well as blood sugar test and it has the ability to display geographical data for community level surveillance. However, it requires a relatively large box accessory and its use of test strips appears to be unsuitable for home healthcare applications due to hygiene problems. (The dipstick is dipped in urine and then inserted in the mat while using a white paper as background and the mat is aligned with the frame, then it is imaged).

Ginardi, R. et al. (in Information And Communication Technology Book Series: Lecture Notes in Computer Science Volume: 8407 Pages: 66-77 Published: 2014) propose a method which includes a framework for color acquisition covering the color management system, color correction, color matching, and quantification. An automated color constancy approach is introduced to provide color correction, and a step-wise linear interpolation is introduced to estimate the urinalysis value. A closed acquisition box is not used. It is claimed that image capturing can be done in any normal lighting condition.

US Patent No 8655009B2, By Chen et al. (Date of Patent: Feb. 18. 2014), "Method and Apparatus for Performing Color-Based Reaction Testing of Biological Materials" discloses a method which includes capturing image of an exposed TS and a reference color chart in an uncalibrated environment. The reference color chart includes a plurality of color blocks arranged in a plurality of test-specific sequences. The method matches these color information, assuming that both color data associated with the reference color chart and the TS are equally impacted by the smart-device camera used and the ambient lighting conditions under which the data was collected, thereby asserting that the method renders calibration unnecessary.

Another article in Lab Chip (Vol 12, No:21, pp:4240-4243, 2012, doi: 10.1039/c21c40741h.) "Point-of-care colorimetric detection with a smartphone", by L.Shen, et.al., and later US Patent Application Publication No. 2015/0055134 A1 by Papautsky et al. (Method and system for analyzing a colorimetric assay, Pub. Date: Feb. 26, 2015) introduce an approach that allows measurements in a wide range of ambient conditions. Instead of directly using the red, green, and blue (RGB) intensities of the color images taken by a smartphone camera, they use chromaticity values to construct calibration curves of analyte concentrations. A calibration method is developed to compensate for measurement errors due to variability in ambient light which is applicable to sun light, fluorescent light, or smartphone LED light.

J. Hong, and BY Chang report development of a smartphone application that digitizes the colors of a colorimetric sensor array, in "Development of the smartphone-based colorimetry for multi-analyte sensing arrays" (Lab Chip. Vol.14 No:10, pp:1725-1732, 2014 doi: 10.1039/c31c51451j. Mar 27, 2014). They report the detection results in terms of color change. Their report focuses on development of a practical application for POC multi-analyte sensing without additional devices. First, the individual positions of the pads are identified, then the colors are digitized based on a correction algorithm, they are converted to concentration values by pre-loaded calibration curves.

S. Cho, ("Smartphone-based, sensitive mPAD detection of urinary tract infection and gonorrhea", Biosensors and Bioelectronics74, pp:601-611, 2015) describe a point-of-care and diagnostic tool that can be used for wider ranges of bacterial concentrations and species, combined with the use of a smartphone. They attempt to evaluate and optimize the antibody conjugation procedure and particle concentration to make the assay adaptable to various disease conditions.

D. Zhang, and Q. Liu, ("Biosensors and bioelectronics on smartphone for portable biochemical detection", Biosensors and Bioelectronics, vol.75, pp: 273 -284, 2016) review the latest development of biosensors and bioelectronics on smartphone for biochemical detections. Smartphone-based optical (Microscopic bio-imaging, Fluorescence, Colorimetric) and Electrochemical (Aerometric, Potentiometric, Impedimetric) methods are summarized in this paper.

A report, "A smartphone algorithm with inter-phone repeatability for the analysis of colorimetric tests", by A. K. Yetisen et.al. (Sensors and Actuators B vol:196 , pp: 156-160 , 2014) presents the results of a study on a smartphone application algorithm with inter-phone repeatability for both Android and iOS operating systems. It is reported that the application transforms the smartphone into a reader to quantify commercial colorimetric urine tests with high accuracy and reproducibility in measuring pH, protein, and glucose with linear responses in the ranges of 5.0-9.0, 0-100 mg/dL and 0-300 mg/dL, respectively.

In another recent article, ("The calibration of cell phone camera-based colorimetric sensor array and its application in the determination of glucose in urine", Biosensors and Bioelectronics vol:74, pp: 1029-1037, 2015), M-Y. Jia et.al. propose an approach that can calibrate the colors obtained with a cellphone camera for the colorimetric sensor array. The ambient light variations, imaging positions and cellphone brands can be compensated via taking the black and white backgrounds of the sensor array as references. Snapshots of the glucose sensor pad were calibrated by the proposed compensation method and the urine samples at different glucose concentrations were discriminated after a hierarchical clustering analysis.

J. Cho, and J. Yoo ("Check4Urine: Smartphone-based Portable Urine-Analysis System", IEMEK J. Embed. Sys. Appl., vol.10 (1) pp: 13-23, Feb. 2015, in Korean) propose a smartphone-based urine-analysis tester and provide three compensation algorithms. The first one improves performance degradation problem robust to various test environments and disturbances, to correct the varying illumination effect, the second is a urine strip detection algorithm robust to edge loss of the object image, and the third is a color decision algorithm based on the pre-processed reference table.

In WO2016/025935A2, a regression model is employed to estimate the level of the total illuminance on the image. Application to dipstick color quantification is also given, wherein an image of the dipstick is processed to estimate a single illuminance level. However, the disclosed approach does not take the factors such as gradient effects, artefacts and effects of color temperature into account.

Images of reagent test strips captured in less controlled ambient conditions, such as point-of-care testing or patient self-monitoring, have high amount of variations. These variations are caused mainly (for example) due to the illumination factors, uncalibrated cameras, etc.

In order to reduce the effect of these variations, most previous art consist of a test strip, a mobile smart-device with a peripheral hardware attachment unit. In other words, they are based on either controlling or correcting for the ambient light conditions, by means of an attached hardware peripheral unit to the smart-device.

Although numerous methods and apparatus have been described in previous art, specific advantages of each of them are offset by disadvantages and shortcomings mainly caused by their dependence on the additional hardware attachment unit to the smart-device. This is due to the fact that such an attachment unit complicates the system, it is not ergonomic, not user friendly, increases the failure rate of the system, introduce reliability problems and occupy large space.

Realizing disadvantages of the ambient light correction attachment, some researchers try to obviete it by employing different algorithms.

However, all previous art of this kind offer deterministic solutions for ambient and positioning problems, while this problem (without using a peripheral hardware attachment unit for positioning and ambient light correction) turns out to be a stochastic one, thus precluding their practicality and effectiveness.

Therefore, previous art (without employing peripheral attachment unit) are prone to have repeatibility problems, and offer weak or no proper solutions to the formation of unexpected shadow(s) on the test strip, reflection from the background and the gradation of light due to improper illumination of the test strip.

None of previous art has a DICOM (Digital Imaging and Communications in Medicine) standard compliant structure.

US patent 2016/048739 A1 discloses training methods for classifiers of test strips images.

US patent WO 2013/116831 A1 discloses a smart phone able to preprocess images of test strips to be analyzed.

Summary

The present invention is defined by the appended claims.

The present invention provides a system for quantifying the metabolites in a biological (such as urine) sample by learning the values corresponding to images of a dataset and estimating the value/label for an unknown sample image. The method of the present invention is based on generating and learning the appearance of the test strip under various conditions.

Therefore, the system disclosed in this patent application eliminates the need for the hardware attachment unit to smart-device.

As the present patent application disclosed herein provides a system consisting merely of a smart-device and a test strip, it brings following advantages: Simple system, ergonomic, and user friendly operation, minimized failure rate of the system, and increased reliability.

Additionally, the system presented herein is an adaptive machine learning algorithm which adapts itself to all positioning and illumination level variations on TS. Accordingly, one obtains accurate, consistent, and standardized quantitative measurement results using the system described herein.

In one embodiment of this invention, image files can be made compliant with Part 10 of the DICOM (Digital Imaging and Communications in Medicine) standard, which enables the integration of the system presented herein with other DICOM compatible medical imaging modalities from different vendors.

Present invention describes a system for quantifying the metabolites (by way of example only) in urine sample by learning the values corresponding to images of a dataset and estimating the value/label for an unknown sample image.

The present invention includes steps of embedding test algorithms for an analyte by means of the test strip, taking a picture of the test strip; processing the picture; displaying the measured values of the analyte on the smartphone and storing these values for future use in at least one of a readable file in the smart device, an external readable file, and a file in a remote server; performing statistical computations, such as a temporal trend of a plurality of the measured analyte values, and correlating the measured values of an analyte to a related selected parameter and displaying correlation coefficients.

It should be noted here that urinalysis application is given by way of example for the description of present patent application only, and present patent application is not limited with this specific clinical use.

The principles described herein may be applied to blood tests, pregnancy tests (to indicate positive or negative presence of hCG), as well as to saliva testing (for example, for the purpose of alcohol consumption testing), or any specific biochemical field in which test strips are employed for diagnostic purposes.

In a preferred embodiment of this invention, a deep learning method based on a convolutional neural network (CNN) model is employed for training. CNN may be leveraged to automatically identify patterns and employ learned visual features to quantify the metabolites in the sample. CNN typically requires inputs to be normalized to the same size and aspect ratio.

In another embodiment of this invention, image features to be used with the classifier such as the pattern and average color of the reagent pads are predefined. This is accomplished by first identifying the test strip or interesting regions on the image, followed by computation of a plurality of descriptors of interesting pixels in the regions identified within the image. These descriptors (image features) are used for training a machine learning algorithm, such as a neural network, support vector machine, naive Bayes, and/or k-nearest neighbor.

In one embodiment of this invention, metabolite quantification problem is reformulated as a classification problem through discretizing the metabolite quantities at pre-defined ranges to classes. In this embodiment, the classes and the corresponding metabolite quantity ranges available on the chart of the dipstick are used as the labels for the classification.

In another embodiment, a regression approach is implemented instead of classification.

Terminology used in this patent application is well known by those who are specialized in the art and can be found in well known text books in the area of digital image processing, and machine learning.

Note that the exemplary description of this invention provides an overview for understanding the nature of the invention and the figures illustrate various embodiments of this invention.

**Detailed Description of the Invention**

The system and method developed to fulfill the objectives of the present invention is illustrated in the accompanying figures, in which:

    **Figure 1** is an example of the test strip.
    **Figure 2** is the flow chart of the biochemical analyzing method.
    **Figure 3** is the data augmentation device.
    **Figure 4** is the examples of simulated illumination conditions.
    **Figure 5** is the flow chart of the pre-processing step.

The biochemical analyzing method of the present invention is based on generating and learning the appearance of a test strip (TS) (dipstick) under various conditions. The method of present invention is different than the existing methods, while the existing methods are based on either controlling or correcting for the ambient lighting conditions.

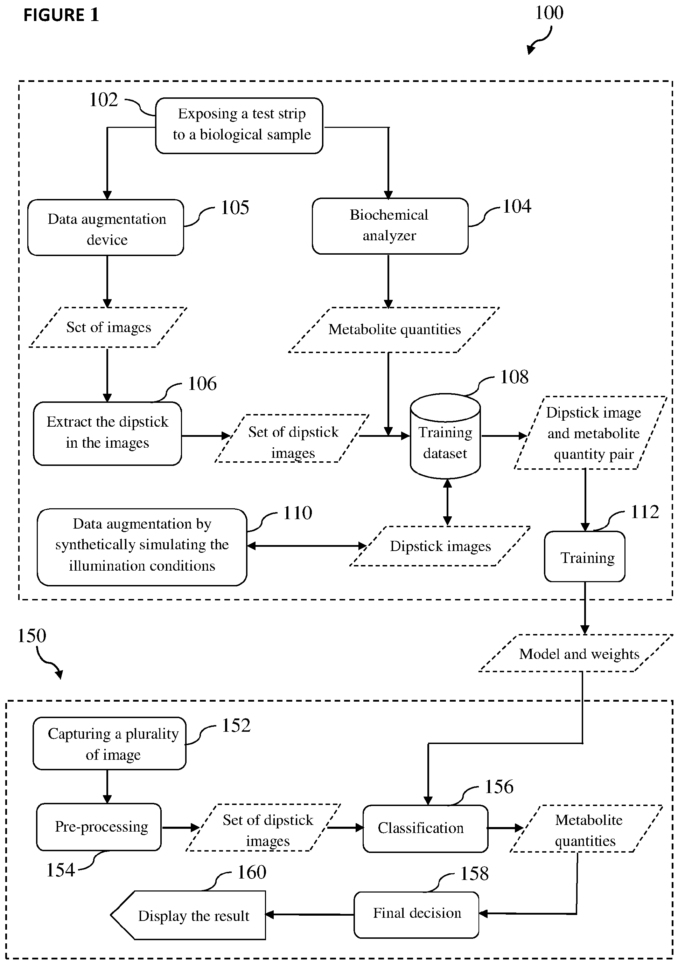

FIG. 1 displays a flowchart of an exemplary method for processing the test strip (TS) to quantify the metabolites. This method consists of two parts: a training part and a testing part. The parameters of a learning model are determined in the training part implemented by the steps of the training method (100) and transferred to the testing part where a testing method (150) is implemented.

It should be noted here that the training method (100) is accomplished once by the vendor at the vendors site. By way of example only, if vendor is a global commercial entity (e.g., a TS manufacturer, an independent organization or company, etc.) besides the sole manufacturer of a specific smart-device model, the training procedure described herein can be repeated using different brands and models of smart-devices. In another example, a medical company or hospital may own the system and perform the training operations on different combinations of smart-devices and test strips (TS). Another example may include plurality of local or international companies as individual owners of the operational system, each performing training operations themselves.

The training method (100) and the testing method (150) are disclosed respectively in the paragraphs below.

The training method (100) for determining parameters of a learning model using a training system comprising a biochemistry analyzer, an optically isolated data augmentation device (300), a computer system having a processor a wired/wireless data communication unit and a storage media, the training method (100) comprises the steps of;

- exposing a test strip (TS) to a biological sample (102),
- measuring metabolite quantities of the test strip (TS) using the biochemistry analyzer (104),
- acquiring a set of images containing the same test strip (TS) by the data augmentation device (300) simulating various ambient lighting conditions using light sources (105),
- extracting the images of the test strip (TS) from the set of the images by the computer system (106),
- saving the extracted images of the test strip (TS) with the corresponding metabolite quantity information measured by the analyzer to a storage media as training samples (108),
- performing one or more data augmentation processes on the training samples using the computer system by the sub steps of (110);

    ▪ retrieving the training samples from the storage media,
    ▪ processing the training samples by simulating at least one of illumination effects selected from a group comprising gradation of light, shadow and reflection of light effect, intensity, color and color temperature of the lighting,
    ▪ saving the processed training samples to the storage media as new training samples,

- training a learning model to determine parameters of the learning model using the training samples stored in the storage media (112),
- transferring the parameters and the learning model by the computer system to a remote server to store them.

The training method (100) is implemented in a training system comprising a biochemistry analyzer; a device (300) for simulating various ambient conditions; a computer system having a wired/wireless data communication unit (such as an Ethernet Card, Bluetooth, WiFi, GSM module, 3G/4G module...), an storage media (such as an internal hard disk of the computer system, an external hard disk, a flask disk, a CD, a cloud based server...), a processor for processing the training samples stored in the storage media; and a remote server for storing the parameters of the learning model.

In one embodiment, the biochemistry analyzer and the data augmentation device (300) can be connected to the computer system via wired or wireless communication channel. In another embodiment, the outputs of the biochemistry analyzer and the data augmentation device can be transferred to the computer system by means of a flash disk, CD...

In the first step of the training method (100), a test strip (TS) is dipped in a biological sample (such as a urine sample) filled in a container (102). The dipping process can be implemented by a user manually. Alternatively, a machine can be used to expose the test strip (TS) to a biological sample.

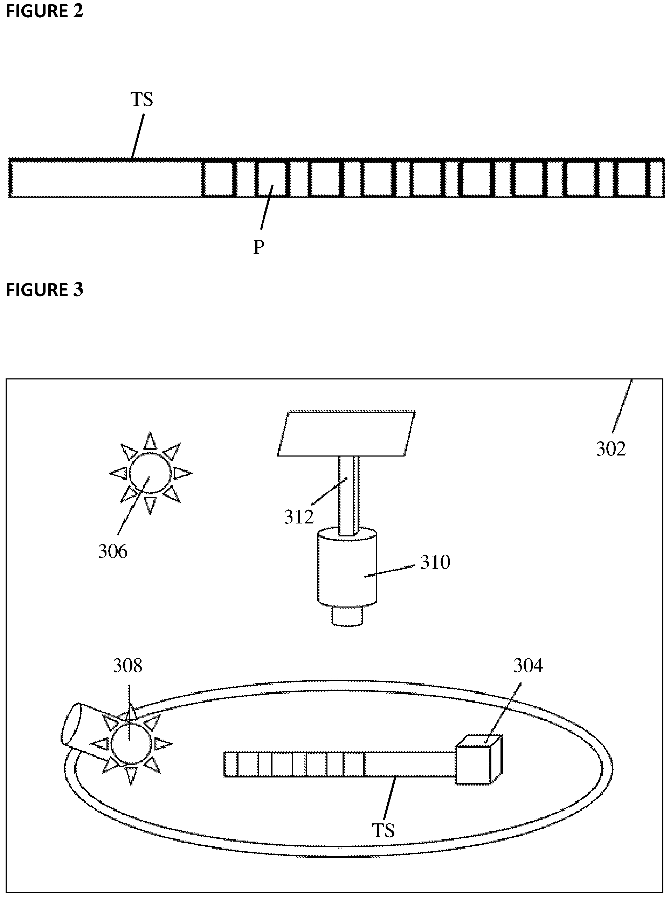

The test strip (TS) is an input sample to the training system. FIG.2 demonstrates a typical test strip (TS) (dipstick) used in urinalysis. This is by way of example only, and there can be plurality of forms of test strips having different number of pads (P) on each of them. In the typical application, the handle of the test strip (TS) is held and the test strip (TS) is dipped into the urine sample filled in a cup. When exposed to urine sample, distinct reactive regions (pads (P)) of the test strip (TS) respond by a change in color or forming a pattern, indicating the amount of metabolite in the sample. For a urine test strip (TS), reactive pads (P) may be sensitive (for example) to the concentration of leukocytes, nitrite, urobilinogen, protein, pH, blood, ketone, bilirubin, glucose, ascorbic acid, etc.

Images of test strips (TS) captured in less controlled ambient lighting conditions, such as point-of-care testing or patient self-monitoring, have high amount of variations. These variations are caused mainly due to the illumination factors. Some commercially available test strips (TS) have their own reference regions for the normalization of the color values and some have spaces between the pads (P), while in some test strips (TS) the spaces between the pads (P) could be used as a reference color.

The dipsticks utilized in the present invention are not part of the invention per se; they are commercially available worldwide (such as Multistix by Siemens, Combur Test by Roche, CombiScreen by Analyticon Biotechnologies, Urocolor by Standard Diagnostics, Uriscan by YD Diagnostics, Mission by Acon Laboratories).

It is assumed here that dipsticks function properly, i.e., they have valid shelf life and they are not reused or compromised by excess humidity which degenerates reagent activity on each pad to indicate false colorimetric results.

In the next step of the training method (100), any reliable existing method to quantify the metabolite quantities can be used to determine a true value for the metabolite quantities (104). As an example application of this invention, the metabolite quantities for the sample test strip (TS) can be measured by a biochemistry analyzer (such as a laboratory urine analyzer, a blood testing device, a saliva testing device...) and serve as a reference of the true value.

Images of the same test strip (TS) are also acquired by a data augmentation device (300), which simulates various ambient conditions, such as the intensity, color or direction of the illumination of the test strip (TS) (105). The device (300) used for image acquisition is presented in FIG. 3 and explained in detail in the subsequent text.

The set of images acquired by the device (300) are processed by the computer system to detect and extract the images of the test strip (TS) (106). The images of the test strip (TS) are detected and extracted by a template matching algorithm (such as the one implemented in ARToolkit (DAQRI, Los Angeles, CA, USA)). In this step, color information of individual pads of the test strip (TS) are evaluated and matched. The set of test strip (TS) images with the corresponding metabolite quantity information obtained in the step (104) are saved to the storage media (108) as training samples.

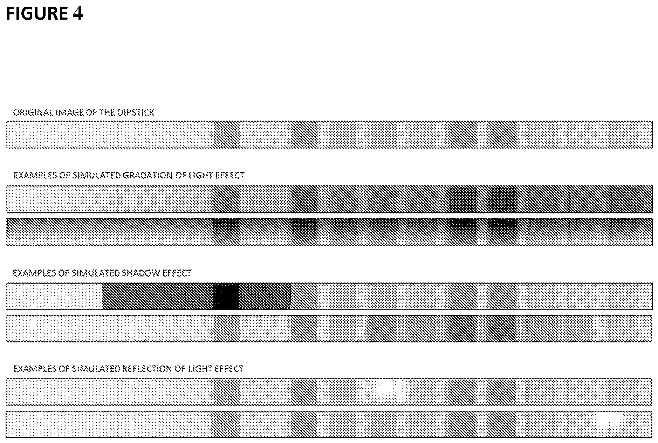

Furthermore one or more data augmentation processes can be performed on the training samples on the computer system to increase the sample size of the training dataset in the storage media (110). In these processes, various illumination conditions are synthetically simulated with random parameters. At each step, a training sample from the dataset in the storage media is retrieved, processed and written back to the dataset as a new entry. The effect of illumination can be a combination of the random directional effects such as gradation of light, shadow, reflection of light, or the global homogeneous effects such as the intensity, color and color temperature of the lighting. The details of the algorithms used are explained in the subsequent paragraphs and sample results are provided in FIG. 4.

A machine learning method is employed to determine a mapping from the test strip images to the metabolite quantities. The training data in the storage media, which consist of sample view of the test strip (TS) and corresponding metabolite quantities, are used to train the computer system to determine the parameters of a learning model (112). The training process can be performed on the computer system or any electronic device.

In a preferred embodiment of this invention, a deep learning method based on a convolutional neural network (CNN) model is employed. CNN may be leveraged to automatically identify patterns and employ learned visual features to quantify the metabolites in the sample. The neural network model processes the input data layer by layer sequentially to calculate a corresponding output. The hyper-parameters of the network model, such as the type, number, order and size of the layers are specific to application and need to be tuned. In this embodiment, the CNN structure consists of six layers, including two convolutional layers, each followed by a max-pooling layer, and two fully-connected layers with Rectified Linear Unit (ReLU) activation neurons. During the training phase, an objective function (such as Euclidean loss), which compares the measured and calculated metabolite quantities for the training set, is minimized by using Stochastic Gradient Descent algorithm. The weights of the network are initialized randomly and derivatives of the objective function with respect to the weights of any layer are calculated using a back-propagation algorithm.

In another embodiment of this invention, image features to be used with the classifier (such as the pattern, or the average value of each color channel of the reagent pads) are predefined, instead of an automatic detection. This is accomplished by first identifying the test strip or interesting regions on the image, followed by computation of a plurality of descriptors of pixels in the regions identified within the image. These descriptors (image features) are used for training a machine learning algorithm, such as a neural network, support vector machine, naive Bayes, and/or k-nearest neighbor.

Once the parameters (such as the weights for a neural network) of the learning model are learned, the model can be used on any smart device (such as a smart phone or tablet PC) to quantify an unknown sample. The output of the training phase (100), consisting of the parameters and the structure of the model are transferred to the testing phase (150), which is executed in a client smart device. The training sample data generation and training processes do not have to be sequential. Instead, the training process may continue while new data are added to the training dataset and the performance of the method is expected to increase with the increase in the number of training samples in the dataset. The size of the training data is not limited since new samples of test strips (TS) can be added by biochemistry laboratories and also the randomization of the data augmentation allows to produce many appearances of the same strip (TS). The improved version of the model by increased number of training data is transferred to the clients as a software update through some communication channel such as the network, internet, memory card, USB connection, or Bluetooth connection and stored in the memory of the smart device.

The testing method (150) for determining metabolite quantities of a test strip (TS) using a testing system comprising; a smart device having a camera, a memory storing the parameters and structure of the learning model determined in the training method (100), a wireless communication module, a screen and a processor, and a remote server storing the parameters and structure of the learning model determined in the training method (100), wherein the smart device and the remote server are connected via a communication network, the testing method (150) comprises the steps of;

- capturing and storing a plurality of images containing a test strip (TS) using the smart device (152),
- pre-processing the images by extracting the images of the test strip (TS) from the captured images and standardizing them in terms of the size and the aspect ratio (154),
- classifying the standardized images of the test strip (TS) using the learning model structure and parameters to assign metabolite values (156),
- combining the values of the test strips (TS) and determining the final metabolite quantities (158),
- displaying the final metabolite quantities on the screen of the smart device (160).

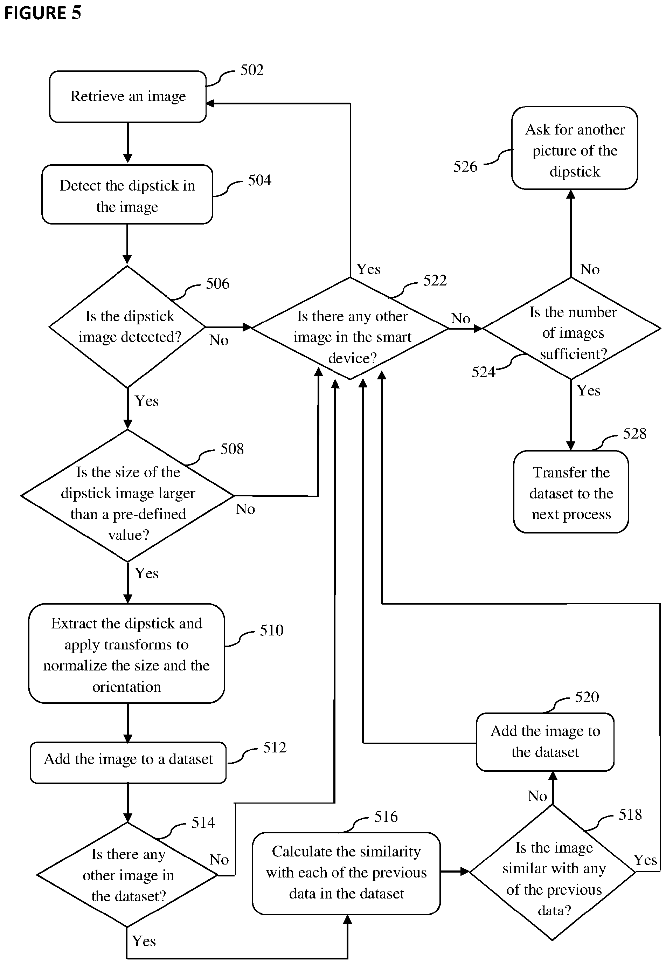

The testing method (150) is executed on client's smart device. A single image, sequence of images or a movie of the test strips (TS) (which is the same type and brand with the test strips (TS) used in the training method (150)) is acquired by the smart device (152) (which is the same type and brand with the smart device used in the device (300)). During the picture taking process (152), the user is guided to take several different images of the test strip (TS). This process simulates the manual quantification scenario where the user randomly rotates the strip to understand the effect of illumination on the appearance of the dipstick. Acquired data can be a single value, or a vector (i.e. RGB) for each pixel in the acquired images. Each of the acquired images are processed in the pre-processing stage (154), which consists of detecting and extracting the test strip (TS) in the images and evaluating the sufficiency of the amount of acquired data under different conditions. A flowchart of an exemplary method for pre-processing the input acquired with the camera is given in FIG.5 and the details of the pre-processing algorithm used are explained in the subsequent paragraphs.

In pre-processing (154), images are standardized in terms of the size and the aspect ratio, and supplied for classification. The learning model structure and parameters retrieved from the training phase are used to classify each picture of the unknown sample to assign metabolite values (156). The values obtained for each picture of the test strip (TS) are combined, by a robust averaging method such as median, cropped mean or mode of the distribution (mode of population), to make a decision on the final metabolite quantities and uncertainties (158). In one embodiment, the mode of the population is used as the final metabolite quantities and the uncertainty is determined as the difference between the maximum and minimum values of the population. The metabolite quantities determined for the given test sample are displayed on the screen (160). Alternatively, the results are transferred to a remote server through a communication channel such as the network, internet, memory card, usb connection, bluetooth connection, or printed on a paper via a printer connected to the smart device via wired or wireless connection, or saved on a storage medium (such as the remote server or memory of the smart device).

Furthermore, test results are made possible by performing statistical computations, such as a temporal trend of a plurality of the measured analyte values, and correlating the measured values of an analyte to a related diagnostic parameter (such as blood pressure) and displaying correlation coefficients.

FIG. 3 shows a block diagram of an exemplary device (300) to generate the images of the test strip (TS) under various conditions. The device (300) is only used for training the machine learning system, for example at the biochemistry laboratory, and is not required at the client (enduser = patient) side.

The device (300) for generating images of the test strip (TS) under various illumination conditions, comprises;

- a shielding layer (302) for isolation from environment,
- a holder (304) for holding the test strip (TS),
- a static light source (306) providing a homogeneous illumination of environment in the shielding layer (302),
- at least one directional light source (308) for illuminating the test strip (TS) attached to the holder (304),
- an imaging means (310) for acquiring a single image, multiple images or a video,
- a positioning device (312) which is adapted to hold the imaging means (310) and adjust distance between the imaging means (310) and the test strip (TS).

The test strip (TS) is placed to the holder (304), which can rotate around three axes by means of three motors rotating in yaw, pitch and roll axis. The motors are driven by a controller (not shown in the figures). In one embodiment, the device (300) has a user interface (such as a keypad or a touch screen) connected to the controller and adapted to take commands from a user for enabling the user to control the rotation of each motor in desired axes. In another embodiment, the device (300) comprises a controller having a memory storing predetermined rotation data, and it sends signals to each motor to rotate them automatically in predetermined axes when the device (300) is started.

The device (300) is optically isolated from ambient in the visible and infrared spectrum by the shielding layer (302). There are two different kinds of illumination sources. The first one is a static light source (306) (such as a diffuse light) having multiple LED sources, which is capable of providing a homogeneous illumination on the test strip (TS) held by the holder (304). The intensity and color temperature of the static light source (306) can be adjusted. This is achieved by adjusting the relative intensity of the multiple LED sources with different color temperatures. In one embodiment, color temperature of the LED sources are controlled via a user interface (such as comprising buttons, a touch screen, or combination thereof) connected to the controller and adapted to take commands from a user. In another embodiment, the controller controls the color temperature of the LED sources in a predetermined range and predetermined time periods, when the device (300) is started.

The second type of illumination source is the directional light source (308). The device can have one or multiple directional light sources (308), which provide illumination effects such as shading, gradient of light or reflection on the strip. In one embodiment, the directional light source (308) is static and the test strip (TS) rotates in three axes to generate illumination effect in various directions. In another embodiment, the directional light source (308) placed on a 360 degree rotating ring to illuminate the test strip (TS), at the center, attached to the holder (304). In another embodiment, the directional light source (308) is fixed and the holder (304) is attached on a rotating table, wherein the table can rotate 360 degrees by means of a motor.

In one embodiment, the directional light source (308) is monochromatic or having a narrow band spectrum. In another embodiment, spectrum of the directional light source (308) consists of a combination of monochromatic spectra. In one embodiment, the intensity and/or the color temperature of the directional light is adjustable.

In an alternative embodiment, at least two directional light sources (308) are used in the device (300). In this embodiment at least one of the directional light source (308) is monochromatic. In another embodiment, each of the directional light is monochromatic or having a narrow band spectrum. In another embodiment, spectrum of monochromatic or narrow band spectrum of each of the directional light is variable. In another embodiment, spectrum of each of the directional light consists of a combination of monochromatic spectra. In one embodiment, the intensity of each of the directional light is adjustable. In one embodiment, the color temperature of each of the directional light is adjustable.

In one embodiment, the static light source (306) is monochromatic or having a narrow band spectrum. In another embodiment, the spectrum of monochromatic or narrow band spectrum of the static light is variable. In one embodiment, the spectrum of the static light consists of a combination of monochromatic spectra.

In one embodiment, the intensity and/or the color temperature of the static light is adjustable.

The imaging means (310) (the same brand and type with smart device used in the testing method (150)) is mounted on the positioning device (312) with adjustable height and acquires a single image, multiple images or video for each different generated view of the test strip (TS). The size of the test strip (TS) on the image is controlled by adjusting the distance between the imaging means (310) and the test strip (TS) using the positioning device (312). Alternatively, the size of the test strip (TS) is controlled by a zoom lens with an adjustable focal length, connected to the imaging means (310). In one embodiment, both positioning device (312) and the adjustable focal length are used in the device (300).

FIG. 4 illustrates exemplary synthetic illumination conditions simulated and applied to an exemplary test strip (TS). Example simulation models for the directional illumination effects are given below. These equations would be applied to each channel (red, green and blue channel) for a multi-channel image (i.e. an RGB color image).

**Gradation of light:**

Given an image of the TS $I(\vec{x})$. a gradient direction vector $\vec{G}$, and a gradient strength value $s$, the resultant image $S(\vec{x})$ of the gradation of light effect is calculated by the equation given in EQN.1.

$$S(\vec{x}) = I(\vec{x}) - s(\vec{G} \cdot \vec{x}) \qquad (\text{EQN.1})$$

where $\vec{x}$ is the spatial position vector.

**Shadow:**

Alternatively, the shadow on the test strip (TS) is modeled as a disc with a random strength located at a random location and size on the image.

Given an image of the TS $I(\vec{x})$, a random location $\vec{r}$, a random radius d, and a strength s, the resultant image $S(\vec{x})$ of the shadow effect is calculated by the equation given in EQN.2.

$$S(\vec{x}) = I(\vec{x}) - s\, H(d - \|\vec{x} - \vec{r}\|) \qquad (\text{EQN.2})$$

where $\|\cdot\|$ defines the L2-norm operator and H denotes the unit step function defined in EQN.3.

$$H(x) = \begin{cases} 1 & x > 0 \\ 0 & x \leq 0 \end{cases} \qquad \text{(EQN.3)}$$

**Reflection of light:**

The reflection of light on the TS is modeled by convolving a spot at a random location on the image with a point spread function, PSF (i.e. Airy disk or Gaussian). The PSF is modelled as a gaussian function.

Given an image of the TS $I(\vec{x})$, a random location $\vec{r}$, a radius d, and a strength s, the resultant image of the reflection of light effect is calculated by the equation given in EQN.4.

$$S(\vec{x}) = I(\vec{x}) + s\,R(\vec{x}) \qquad \text{(EQN.4)}$$

where the function $R(\vec{x})$ is defined in EQN.5.

$$R(\vec{x}) = PSF(\vec{x}) * H(d - \|\vec{x} - \vec{r}\|) \qquad \text{(EQN.5)}$$

where H denotes the unit step function defined in EQN.3. The PSF is the point spread function in Gaussian form as given in EQN.6.

$$PSF(\vec{x}) = exp(-\|\vec{x}\|^2 / 2\sigma^2) \qquad \text{(EQN.6)}$$

where σ parameter corresponds to the standard deviation of the normal distribution which controls the amount of spread of the reflection spot on the image.

Additionally, the size and orientation of the test strip (TS) can also be simulated on a computer by applying affine transformations to the images.

FIG. 5 displays a flowchart of an exemplary method for pre-processing the input data which is acquired by the client's smart device. The pre-processing step (154) comprises the sub-steps of;

- retrieving an image stored in the smart device (502),
- detecting the image of the test strip (TS) in the retrieved image (504),
- querying if the image of the test strip (TS) is detected (506),
  - if no, querying if there any other image stored in the smart device (522),

    • if yes, turning back to the image retrieving step (502) to retrieve another image,
    • if no, querying the number of image of the test strip (TS) in a dataset to determine if it exceeds a predefined value (524),

      ◦ if no, turning back to the image capturing step (152) and asking for another image (526),
      ◦ if yes, transferring the images in the dataset to the next process (528),

- querying if the size of the image is in predefined size limits (508),
  - if no, turning back to the step of querying image in the smart device (522),
- extracting the image of the test strip (TS) from the retrieved image and applying an affine transform to the extracted image to normalize the size and the orientation of the image of the test strip (TS) (510),
- adding the image to the dataset in the memory (512),
- querying the number of images in the dataset whether there is another image or not (514),
  - if no, turning back to the step of querying image in the smart device (522),
- calculating the similarity measure between the image and previous images stored in the dataset (516),
- querying if the image is similar with any of the images stored in the dataset considering the maximum value of the similarity (518),
  - if no, appending the image to the dataset (520),
- turning back to the step of querying image in the smart device (522),

In pre-processing (154), each image captured and stored by the smart device in 152 is processed one by one as a different sample.

Firstly, an image including the test strip (TS) is retrieved from the memory storing a plurality of the images captured by the smart device (502). The test strip (TS) in the image is detected by a template matching algorithm (such as given in ARToolkit) (504). In this detecting step, color information of individual pads (P) of a test strip (TS) are evaluated and matched and an affine transform is obtained.

If the image of the test strip (TS) is detected (506) and size of the test strip (TS) is in the predefined suitable range (508), the image of the test strips (TS) are cropped and the affine transform obtained in 504 is applied to normalize the size and the orientation of the test strip (TS) image (510). Then, the processed image is incorporated into a dataset stored in the memory of the smart device (512).

For each new sample incorporated into the dataset, a similarity measure (i.e., based on L2 norm or mutual information) between the images in the dataset is calculated (516). The step 518 checks the non-similarity of the new sample with the images in the dataset by considering the maximum value of the similarity with any of the sample in the dataset. If this value is lower than a pre-defined threshold, the sample is appended to the dataset in 520.

If any one of the query in the steps of 506, 508, 514 and 518 fails, a control step (522) querying a dataset in the memory of the smart device is initiated. If there is another image in the dataset, it is retrieved for processing. Otherwise, another control step (524) is initiated to query the number of image samples of the dipstick in the dataset to determine if it exceeds a predefined value. If sufficient number of samples is collected, the set of dipstick images in the dataset is transferred to the next process (528). Otherwise, the user is warned through the smart device's screen and/or speaker to capture one or more images of the test strip (TS) (526).

In one embodiment, if it is determined in the step of 508 that the size of test strips (TS) captured by the smart device are not within a pre-defined range (514), the user may be warned to get closer or move away to obtain an image between the predefined size limits in the steps of (526).

In a preferred embodiment of this invention, image files can be made compliant with Part 10 of the DICOM (Digital Imaging and Communications in Medicine) standard. This standard is for handling, storing, printing, and transmitting information in medical imaging, which enables the integration of medical imaging devices from different vendors as they come with DICOM Conformance Statements. The standard has been widely adopted and in use by hospitals worldwide and part 10 of the standard presents a file format for the distribution of images.

**Claims**

1. A testing method (150) for determining metabolite quantities of a test strip (TS) using a testing system comprising: a smart device having a camera, a wireless communication module, a screen, a processor and a memory storing the parameters and structure of a learning model determined in the training method (100) **comprising the steps** of:

    - exposing a test strip (TS) to a biological sample (102),
    - measuring metabolite quantities of the test strip (TS) using the biochemistry analyzer (104),
    - acquiring a set of images containing same test strip (TS) by the data augmentation device (300) simulating ambient lighting conditions using light sources (105),
    - extracting the images of the test strip (TS) from the set of the images by the computer system (106),
    - saving the extracted images of the test strip (TS) with the corresponding metabolite quantity information measured by the analyzer to a storage media as training samples (108),
    - training a learning model to determine parameters of the learning model using the training samples stored in the storage media by the computer system (112),
    - transferring the parameters and the learning model to a remote server via the wireless communication unit to store them, the testing method (150) **comprising the steps** of:

        - capturing and storing a plurality of images containing a test strip (TS) using the smart device (152),
        - pre-processing the images by extracting the images of the test strip (TS) from the captured images (154),
        - classifying the standardized images of the test strip (TS) using the learning model structure and parameters to assign metabolite values (156),
        - combining the values of the test strips (TS) and determining the final metabolite quantities (158),
        - displaying the final metabolite quantities on the screen of the smart device (160) and **characterized by** standardizing the pre-processed images in the step of "pre-processing the images by extracting the images of the test strip (TS) from the captured images (154)" in terms of the size and the aspect ratio.

**EP 3 612 963 B1**

**Patentansprüche**

1. Testverfahren (150) zur Bestimmung von Metabolitmengen eines Teststreifens (TS) unter Verwendung eines Testsystems, umfassend Folgendes:

   eine Smart-Vorrichtung mit einer Kamera,
   einem drahtlosen Kommunikationsmodul, einem Bildschirm, einem Prozessor und einem Speicher, der die Parameter und die Struktur eines Lernmodells speichert, das in dem Trainingsverfahren (100) bestimmt wurde, **das die folgenden Schritte umfasst:**

   - Aussetzen eines Teststreifens (TS) zu einer biologischen Probe (102),
   - Messen von Metabolitmengen des Teststreifens (TS) mit dem Biochemie-Analysegerät (104),
   - Erfassen eines Satzes von Bildern, die denselben Teststreifen (TS) enthalten, durch die Datenvergrößerungsvorrichtung (300), die Umgebungslichtbedingungen unter Verwendung von Lichtquellen (105) simuliert,
   - Extrahieren der Bilder des Teststreifens (TS) aus dem Satz der Bilder durch das Computersystem (106),
   - Speichern der extrahierten Bilder des Teststreifens (TS) mit den entsprechenden vom Analysegerät gemessenen Metabolitmengeninformationen auf einem Speichermedium als Trainingsproben (108),
   - Trainieren eines Lernmodells zur Bestimmung von Parametern des Lernmodells unter Verwendung der in den Speichermedien gespeicherten Trainingsproben durch das Computersystem (112),
   - Übertragen der Parameter und des Lernmodells an einen entfernten Server über die drahtlose Kommunikationseinheit, um sie zu speichern, wobei das Testverfahren (150) **die folgenden Schritte umfasst:**

     - Erfassen und Speichern einer Vielzahl von Bildern, die einen Teststreifen (TS) enthalten, unter Verwendung der Smart-Vorrichtung (152),
     - Vorverarbeiten der Bilder durch Extrahieren der Bilder des Teststreifens (TS) aus den erfassten Bildern (154),
     - Klassifizieren der standardisierten Bilder des Teststreifens (TS) unter Verwendung der Lernmodellstruktur und der Parameter, um Metabolitenwerte (156) zuzuordnen,
     - Kombinieren der Werte der Teststreifen (TS) und Bestimmen der endgültigen Metabolitmengen (158),
     - Anzeigen der endgültigen Metabolitenmengen auf dem Bildschirm der Smart-Vorrichtung (160) und **gekennzeichnet durch** Standardisieren der vorverarbeiteten Bilder im Schritt "Vorverarbeiten der Bilder durch Extrahieren der Bilder des Teststreifens (TS) aus den erfassten Bildern (154)" in Bezug auf die Größe und das Seitenverhältnis.

**Revendications**

1. Procédé de test (150) pour déterminer les quantités de métabolites d'une bande de test (TS) en utilisant un système de test comprenant :

   un dispositif intelligent ayant un caméra,
   un module de communication sans fil, un écran, un processeur et une mémoire stockant les paramètres et la structure d'un modèle d'apprentissage déterminé dans le procédé d'entraînement (100) **comprenant les étapes suivantes:**

   - l'exposition d'une bande de test (TS) à un échantillon biologique (102),
   - la mesure des quantités de métabolites de la bande de test (TS) en utilisant l'analyseur biochimique (104),
   - l'acquisition d'un ensemble d'images contenant la même bande de test (TS) par le dispositif d'augmentation des données (300) en simulant les conditions d'éclairage ambiant en utilisant des sources lumineuses (105),
   - l'extraction des images de la bande de test (TS) de l'ensemble des images par le système informatique (106),
   - la sauvegarde des images extraites de la bande de test (TS) avec les informations correspondantes sur la quantité de métabolites mesurée par l'analyseur sur un support de stockage en tant qu'échantillons d'entraînement (108),
   - l'entraînement d'un modèle d'apprentissage pour déterminer les paramètres du modèle d'apprentissage en utilisant les échantillons d'entraînement stockés dans les supports de stockage par le système informatique (112),

- le transfert des paramètres et du modèle d'apprentissage vers un serveur distant via l'unité de communication sans fil pour les stocker, le procédé de test (150) **comprenant les étapes suivantes:**

- la capture et le stockage d'une pluralité d'images contenant une bande de test (TS) en utilisant le dispositif intelligent (152),
- le prétraitement des images en extrayant les images de la bande de test (TS) des images capturées (154),
- la classification des images standardisées de la bande de test (TS) en utilisant la structure et les paramètres du modèle d'apprentissage pour attribuer des valeurs aux métabolites (156),
- la combinaison des valeurs des bandes de test (TS) et la détermination des quantités finales de métabolites (158),
- l'affichage des quantités finales de métabolites sur l'écran du dispositif intelligent (1600) et **caractérisé par** la standardisation des images prétraitées dans l'étape de "prétraitement des images en extrayant les images de la bande de test (TS) des images capturées (154)" en termes de taille et de format.

**FIGURE 1**

100

102 — Exposing a test strip to a biological sample

105 — Data augmentation device

104 — Biochemical analyzer

Set of images

Metabolite quantities

106 — Extract the dipstick in the images

Set of dipstick images

108 — Training dataset

Dipstick image and metabolite quantity pair

110 — Data augmentation by synthetically simulating the illumination conditions

Dipstick images

112 — Training

150

Model and weights

152 — Capturing a plurality of image

Pre-processing

154

Set of dipstick images

156 — Classification

Metabolite quantities

160 — Display the result

158 — Final decision

**FIGURE 2**

**FIGURE 3**

**FIGURE 4**

ORIGINAL IMAGE OF THE DIPSTICK

EXAMPLES OF SIMULATED GRADATION OF LIGHT EFFECT

EXAMPLES OF SIMULATED SHADOW EFFECT

EXAMPLES OF SIMULATED REFLECTION OF LIGHT EFFECT

**FIGURE 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6514461 B1, Lappe
- US 5408535 A, Howard
- US 8797536 B2, D. S. Lee
- WO 2015042523 A1
- US 20120244624 A1
- US 8506901 B, Chen
- US 20150359458 A1, D. Erickson

- WO 2015038717 A1, N. Agarwal
- US 8655009 B2, Chen
- US 20150055134 A1, Papautsky
- WO 2016025935 A2
- US 2016048739 A1
- WO 2013116831 A1

**Non-patent literature cited in the description**

- **GINARDI, R. et al.** *Information And Communication Technology Book Series: Lecture Notes in Computer Science,* 2014, vol. 8407, 66-77
- *Lab Chip,* 2012, vol. 12 (21), 4240-4243
- Development of the smartphone-based colorimetry for multi-analyte sensing arrays. *Lab Chip.,* 27 March 2014, vol. 14 (10), 1725-1732
- **S. CHO.** Smartphone-based, sensitive mPAD detection of urinary tract infection and gonorrhea. *Biosensors and Bioelectronics,* 2015, vol. 74, 601-611
- **D. ZHANG ; Q. LIU.** Biosensors and bioelectronics on smartphone for portable biochemical detection. *Biosensors and Bioelectronics,* 2016, vol. 75, 273-284

- **A. K. YETISEN.** A smartphone algorithm with inter-phone repeatability for the analysis of colorimetric tests. *Sensors and Actuators B,* 2014, vol. 196, 156-160
- The calibration of cell phone camera-based colorimetric sensor array and its application in the determination of glucose in urine. *Biosensors and Bioelectronics,* 2015, vol. 4, 1029-1037
- **J. CHO ; J. YOO.** Check4Urine: Smartphone-based Portable Urine-Analysis System. *IEMEK J. Embed. Sys. Appl.,* February 2015, vol. 10 (1), 13-23